# EUROPÄISCHE PATENTSCHRIFT

(11) **EP 2 193 243 B1**
(45) Veröffentlichungstag und Bekanntmachung des Hinweises auf die Patenterteilung: **16.03.2016**
(21) Anmeldenummer: 08804961.4
(22) Anmeldetag: 01.10.2008
(51) Int. Cl.: E04C 2/04, A61L 9/20, D21H 13/00

(54) **BAUPLATTE AUF BASIS VON GIPS**
STRUCTURAL PANEL MADE OF PLASTER
PANNEAU DE CONSTRUCTION SUR LA BASE DE PLÂTRE

(30) Priorität: 01.10.2007 EP 07117629
(43) Veröffentlichungstag der Anmeldung: 09.06.2010
(73) Patentinhaber: Knauf Gips KG, 97346 Iphofen (DE)
(72) Erfinder: HUMMEL, Hans-Ulrich, 97346 Iphofen (DE)
(74) Vertreter: von Kreisler Selting Werner - Partnerschaft von Patentanwälten und Rechtsanwälten mbB
(86) Internationale Anmeldenummer: PCT/EP2008/063140
(87) Internationale Veröffentlichungsnummer: WO 2009/047184

(56) Entgegenhaltungen:
- EP-A- 0 590 477
- WO-A-2006/059699
- DE-A1- 10 149 414
- DE-A1-102006 009 847
- DE-B3-102006 022 083
- DE-U1-202006 014 693
- US-A1- 2007 298 235

## Beschreibung

Die vorliegende Erfindung betrifft eine Bauplatte auf Basis von Gips und Verfahren zu ihrer Herstellung.

Bauplatten auf Basis von Gips werden im großen Umfang insbesondere im Innenausbau von Gebäuden eingesetzt, insbesondere zur Herstellung von Decken und Wänden.

Die übliche Bauplatte auf Basis von Gips ist eine sogenannte Gipskartonplatte, bei der die Gipsmasse beidseitig mit einer Kartonschicht abgedeckt ist. Es sind verschiedene Spezialplatten bekannt, die beispielsweise statt einer Abdeckung mit einem Karton eine Abdeckung mit einem Vlies aufweisen. Solche sind beispielsweise in der EP 0 755 903 A1 beschrieben.

Bei der Herstellung einer Gipsplatte mit einem Vlies dringt der Gipsbrei in das Gewebe ein; ist dies nicht der Fall, wird keine hinreichende Trockenhaftung zwischen dem Gips und dem Vlies erreicht.

Die steigenden Anforderungen an die Wärmedämmung von Gebäuden führen dazu, dass die Gebäude annähernd "luftdicht" gegen die Außenwelt abgeschlossen sind und sich daher die Luftwechselraten in den Räumen stark reduziert haben. Hierdurch werden Belastungen durch Gerüche und Luftschadstoffe zu einem vermehrten Problem.

WO 2006/059699 betrifft ein Vlies für eine Gipsplatte, das aus Glasfasern besteht und mit Titanoxid beschichtet sein kann. Titanoxide sind photokatalytisch aktiv, d.h. unter Anregung mit Licht einer geeigneten Wellenlänge entsteht ein reaktives Material, das über einen radikalischen Mechanismus, z.B. mit Luftschadstoffen reagieren kann.

Eigene Untersuchungen haben gezeigt, dass bei der Herstellung entsprechender Platten große Mengen des Titandioxids vom Gipsbrei umschlossen werden und daher keine photokatalytische Aktivität mehr aufweisen.

Aufgabe der vorliegenden Erfindung war es, ein Verfahren bereit zu stellen, mit dem verbesserte Gipsplatten mit einer Titandioxidbeschichtung erhalten werden.

Gelöst wird die Aufgabe durch ein Verfahren zur Herstellung einer Bauplatte, wobei
- ein Glasfaservlies mit einer Beschichtung versehen wird, die ein photokatalytisch aktives Titandioxid enthält,
- das beschichtete Glasfaservlies mit pulverförmigem Füllstoff beladen wird
- das beschichtete Glasfaservlies dann mit einem abbindefähigen Calciumsulfatgemisch beladen wird
- anschließend mit einem unbeschichteten Glasfaservlies abgedeckt wird
- nach dem Beginn des Abbindens geschnitten und getrocknet wird.

Erfindungsgemäß handelt es sich um eine Bauplatte mit einer beidseitigen Glasfaservliesabdeckung, wobei zumindest eine Seite eine Beschichtung aufweist, die auf der Außenseite photokatalytisch aktives Titandioxid enthält.

Photokatalytische Materialien sind Halbleiter, bei denen unter Lichteinwirkung Elektronen-Loch-Paare entstehen, welche an der Oberfläche mit diversen Luftbestandteilen Redox-Reaktionen hervorrufen können.

Als photokatalytisch aktives Titandioxid wird bevorzugt ein Titandioxid eingesetzt, wie es in der EP 1 178 011 A1, EP 1 254 863 und insbesondere in der WO 2005/108505 beschrieben ist.

Bevorzugt wird ein Titandioxid verwendet, dass modifiziert ist, um die Bandlücke von 3 eV zu verringern.

Typische Gehalte des photokatalytisch aktiven Titandioxids im Glasfaservlies betragen bis zu 25 Gew.-%.

Als besonders geeignete Materialien für den Füllstoff haben sich Anhydrit (wasserfreies CaSO₄) und Kalksteinmehl (CaCO₃) erwiesen. Geeignete Korngrößen für den Füllstoff liegen im Bereich von 1 bis 63 µm, vorzugsweise 3 bis 10 µm.

Gegenstand der Erfindung ist auch eine Bauplatte, die durch das erfindungsgemäße Verfahren erhältlich ist.

Gegenstand der Erfindung ist weiterhin die Verwendung der erfindungsgemäßen Bauplatte zur Verringerung von Luftschadstoffen und Gerüchen in Innenräumen.

Die Erfindung wird durch die nachfolgenden Beispiele näher erläutert.

### Beispiel 1

Die bestimmungsgemäße Bauplatte wird auf einer konventionellen Gipsplattenanlage hergestellt. Dazu wird ein Sichtsseitenvlies auf eine Fördereinrichtung abgerollt. Das Sichtseitenvlies ist im Kern aus einem Gemisch aus z.B. 5 Gew.% CaCO₃ und 7 Gew.% CaSO₄ gefüllt und die Außenseite enthält ein Coating aus 20 - 25 Gew.% photokatalytischem TiO₂.

Das Sichtseitenvlies wird mit einem Stuckgipsbrei im Mischerbereich der Gipsplattenanlage beladen. Durch Rütteleinrichtungen wird der Stuckgipsbrei flächig verteilt und zugleich in das Sichtseitenvlies eingerüttelt. Vor einer Extrudierwalze wird das Rückseitenvlies auf die Stuckgipsbreioberfläche geführt. Das Rückseitenvlies entspricht dem Sichtseitenvlies, enthält aber kein TiO₂-Coating.

Im Anschluss an die Extrudierwalze folgt ein bewegtes Abbindeband. Hier erfolgt die Reaktion des Stuckgipses mit Wasser zu Gips. Nach der Abbindereaktion wird der Strang per Schere vereinzelt, gewendet, damit die Sichtseite oben liegt, und in einem Rollentrockner getrocknet.

### Beispiel 2: Deckenkonstruktionen in Rasterbauweise mit Einlegemontage

Hierzu werden quadratische oder rechteckige Platten aus großformatigen Platten herausgeschnitten; bevorzugte Maße ~ 30x30 oder 40x40 cm. Diese Elemente werden zur Einlegemontage in Rasterdecken eingesetzt, ohne dass eine weitere Oberflächenbehandlung oder Oberflächenbeschichtung notwendig ist.

### Beispiel 3: Wandsysteme

Eine weitere Anwendung betrifft die Konstruktion kompletter Wandsysteme im Nicht-Sichtbereich oder im Industriebereich. Hier ist wesentlich, dass außer Fugenverspachtelungen keine weiteren Oberflächenveränderungen in Form von Beschichtungen, Anstrichen etc. vorgenommen werden.

## Patentansprüche

1. Verfahren zur Herstellung einer Bauplatte mit einer beidseitigen Glasfaservliesabdeckung wobei zumindest eine Seite eine Beschichtung aufweist, die auf der Außenseite photokatalytisch aktives Titandioxid enthält, wobei,
- ein Glasfaservlies mit einer Beschichtung versehen wird, die ein photokatalytisch aktives Titandioxid enthält,
- das beschichtete Glasfaservlies mit pulverförmigem Füllstoff beladen wird,
- das beschichtete Glasfaservlies dann mit einem abbindefähigen Calciumsulfatgemisch beladen wird
- anschließend das Calciumsulfatgemisch mit einem unbeschichteten oder beschichteten Glasfaservlies abgedeckt wird, und
- nach dem Beginn des Abbindens die Bauplatte geschnitten und getrocknet wird.

2. Verfahren nach Anspruch 1, **dadurch gekennzeichnet, dass** die Beschichtung bis 90 Gew.-% photokatalytisch aktives Titandioxid enthält.

3. Verfahren nach Anspruch 1 oder 2, **dadurch gekennzeichnet, dass** der inerte Füllstoff ausgewählt wird aus Calciumsulfat-Anhydrit, Kalksteinmehl oder Mischungen davon.

4. Verfahren nach mindestens einem der Ansprüche 1 bis 3, **dadurch gekennzeichnet, dass** die Beschichtung ein Flächengewicht von 280 bis 330 g/m² hat.

5. Bauplatte erhältlich durch ein Verfahren nach einem der Ansprüche 1 bis 4.

6. Verwendung einer Bauplatte nach Anspruch 5 zur Verringerung von Luftschadstoffen und Gerüchen in Innenräumen.

## Claims

1. A process for producing a building board with a glass fiber mat covering on both sides thereof, wherein at least one side has a coating comprising photocatalytically active titanium dioxide on the outside, wherein
- a glass fiber mat is provided with a coating comprising a photocatalytically active titanium dioxide;
- the coated glass fiber mat is loaded with a powdery filler;
- the coated glass fiber mat is then loaded with a settable calcium sulfate mixture;
- the calcium sulfate mixture is subsequently covered by an uncoated or coated glass fiber mat; and
- after the start of setting, the building board is cut and dried.

2. The process according to claim 1, **characterized in that** said coating comprises up to 90% by weight of photocatalytically active titanium dioxide.

3. The process according to claim 1 or 2, **characterized in that** the inert filler is selected from calcium sulfate anhydride, limestone powder, or mixtures thereof.

4. The process according to at least one of claims 1 to 3, **characterized in that** said coating has a weight per unit area of from 280 to 330 g/m².

5. A building board obtainable by a process according to any of claims 1 to 4.

6. Use of a building board according to claim 5 for reducing air pollutants and smells in interior rooms.

## Revendications

1. Procédé de fabrication d'un panneau de construction, comprenant un revêtement biface de non-tissé de fibres de verre, au moins une face comportant un revêtement qui contient du côté extérieur du dioxyde de titane photo-catalytiquement actif,
- un non-tissé de fibres de verre étant pourvu d'un revêtement contenant un dioxyde de titane photo-catalytiquement actif,
- le non-tissé de fibres de verre pourvu du revêtement étant chargé avec une charge pulvérulente,
- le non-tissé de fibres de verre pourvu du revêtement étant ensuite chargé avec un mélange de sulfate de calcium durcissable,
- ensuite, le mélange de sulfate de calcium est recouvert d'un non-tissé de fibres de verre pourvu ou non d'un revêtement, et
- le panneau de construction est séché et découpé après que le durcissement a commencé.

2. Procédé selon la revendication 1, **caractérisé en ce que** le revêtement contient 90% en poids de dioxyde de titane photo-catalytiquement actif.

3. Procédé selon la revendication 1 ou la revendication 2, **caractérisé en ce que** la charge inerte est choisie parmi l'anhydrite de sulfate de calcium, la poudre de calcaire, ou des mélanges de ceux-ci.

4. Procédé selon l'une au moins des revendications 1 à 3, **caractérisé en ce que** le revêtement présente un grammage de 280 à 330 g/m².

5. Panneau de construction pouvant être obtenu par un procédé selon l'une quelconque des revendications 1 à 4.

6. Utilisation d'un panneau de construction selon la revendication 5 pour réduire les polluants atmosphériques et les odeurs dans des espaces intérieurs.
